# EUROPEAN PATENT APPLICATION

(11) **EP 4 296 280 A1**
(43) Date of publication of application: **27.12.2023**
(21) Application number: 22180264.8
(22) Date of filing: 21.06.2022
(51) Int. Cl.: C07K 16/28, A61P 35/00, A61K 39/395

(54) **CD123 AND CD200 AS MARKERS FOR THE DIAGNOSIS AND IMMUNE-ERADICATION OF LEUKEMIC STEM CELLS (LSCS)**

(71) Applicant: F. Hoffmann-La Roche AG, 4070 Basel (CH); Ludwig-Maximilians-Universität München, 80539 München (DE)
(72) Inventor: Bergér, Daniel, 80337 Munich (DE); Brinkmann, Ulrich, 82377 Penzberg (DE); Dickopf, Steffen, 82377 Penzberg (DE); Klein, Christoph, 80337 Munich (DE)
(74) Representative: Krauss, Jan

(57) **Abstract**

The present invention relates to antigen binding molecules that are at least bispecific and specifically bind to CD200 and CD123 on the cellular surface of leukemic stem cells (LSCs). The present invention further relates to a method for identifying such antigen binding molecules and the use of the antigen binding molecules for the diagnosis and treatment of leukemia, such as AML or CML, and in particular pediatric forms thereof.

## Description

The present invention relates to antigen binding molecules that are at least bispecific and specifically bind to CD200 and CD123 on the cellular surface of leukemic stem cells (LSCs). The present invention further relates to a method for identifying such antigen binding molecules and the use of the antigen binding molecules for the diagnosis and treatment of leukemia, such as AML or CML, and in particular pediatric forms thereof.

### Background of the invention

Cancer is a major lethal disease for humans and is caused by physiologically uncontrolled cell proliferation which affects normal physiological conditions of human body resulting in serious pathological reactions often leading to death. Although tremendous efforts on cancer studies and treatments have been made, presently, cancer is still the major cause of death to humans. There are multiple approaches to treat cancer patients including surgery, radiation therapy and chemotherapy.

Despite major advances in risk-adapted chemotherapy, the therapy of acute myeloid leukemia (AML) remains challenging (Döhner H, Estey E, Grimwade D, Amadori S, Appelbaum FR, Büchner T, et al. Diagnosis and management of AML in adults: 2017 ELN recommendations from an international expert panel. Blood. 2017;129: 424-447. The relative 5-year survival probability is only 30% (Acute Myeloid Leukemia - cancer stat facts. [cited 6 Dec 2021]. Available: https://seer.cancer.gov/statfacts/html/amyl.html). Dormant "leukemic stem cells" (LSC) can survive a cytotoxic therapy and thus be the origin of a relapse (Shlush LI, Mitchell A, Heisler L, Abelson S, Ng SWK, Trotman-Grant A, et al. Tracing the origins of relapse in acute myeloid leukaemia to stem cells. Nature. 2017;547: 104-108). Targeted immunotherapy can be an effective option for eliminating chemoresistant leukemic stem cells.

In acute lymphoblastic B-cell leukemia (B-ALL), so-called "bispecific T-cell-engaging antibody constructs" and "chimeric antigen receptor (CAR)" T cells have been used with some success (Mohanty R, Chowdhury CR, Arega S, Sen P, Ganguly P, Ganguly N. CAR T cell therapy: A new era for cancer treatment (Review). Oncol Rep. 2019;42: 2183-2195; Halford Z, Coalter C, Gresham V, Brown T. A Systematic Review of Blinatumomab in the Treatment of Acute Lymphoblastic Leukemia: Engaging an Old Problem With New Solutions. Ann Pharmacother. 2021;55: 1236-1253).

With CD19, a target protein is present that is strongly overexpressed on the leukemic B cells, while healthy non-B cells are negative for the marker (Ghorashian S, Pule M, Amrolia P. CD19 chimeric antigen receptor T cell therapy for haematological malignancies. Br JHaematol. 2015;169: 463-478). Because CD19 is expressed on normal B cells as well, normal B-lineage cells are also eliminated after CD19-CAR-T infusion. This phenomenon is typically called an "on-target off-tumor effect." Subsequent B-cell aplasia results in long-lasting hypogammaglobulinemia, and intermittent immunoglobulin replacement is occasionally required to prevent severe infections.

For AML, nevertheless, the "ideal" target does not yet exist. All previously known targets overexpressed on AML stem cells, such as CD33, CD123 or CLL-1 can also be found on other and healthy cells. Therapeutic approaches against some of these targets also led to severe on-target-off leukemia toxicity (Kenderian SS, Ruella M, Shestova O, Klichinsky M, Aikawa V, Morrissette JJD, et al. CD33-specific chimeric antigen receptor T cells exhibit potent preclinical activity against human acute myeloid leukemia. Leukemia. 2015;29: 1637-1647; Tashiro H, Sauer T, Shum T, Parikh K, Mamonkin M, Omer B, et al. Treatment of Acute Myeloid Leukemia with T Cells Expressing Chimeric Antigen Receptors Directed to C-type Lectin-like Molecule 1. Mol Ther. 2017;25: 2202-2213; Gill S, Tasian SK, Ruella M, Shestova O, Li Y, Porter DL, et al. Preclinical targeting of human acute myeloid leukemia and myeloablation using chimeric antigen receptor-modified T cells. Blood. 2014;123: 2343-2354).

Leukemia stem cells (LSCs) in AML play important roles in leukemia initiation, progression, and were considered to be the root of chemotherapeutic drug resistance and disease relapse. The identification and targeting LSCs depends on membrane markers like CD34, CD38, CD123, TIM3, CD25, CD32 and CD96. In addition, the transcription factors are also therapeutic targets in eradicating LSCs, such as histone deacetylases (HDACs), NF-κB, HIF-1α and β-catenin. Besides membrane markers and transcription factors, intracellular reactive oxygen species (ROS), telomerase and microRNAs were identified to be new targets for ablating LSCs in AML (Ding Y, Gao H, Zhang Q. The biomarkers of leukemia stem cells in acute myeloid leukemia. Stem Cell Investig. 2017;4:19. Published 2017 Mar 2. doi:10.21037/sci.2017.02.10).

In acute myeloid leukemia (AML), leukemic stem cells (LSCs) underlie mortality but are difficult to isolate due to their low abundance and high similarity to healthy hematopoietic stem cells (HSCs) (Velten, L., Story, B.A., Hernandez-Malmierca, P. et al. Identification of leukemic and pre-leukemic stem cells by clonal tracking from single-cell transcriptomics. Nat Commun 12, 1366 (2021). https://doi.org/10.1038/s41467-021-21650-1).

Haubner, S. et al. (in: Coexpression profile of leukemic stem cell markers for combinatorial targeted therapy in AML Leukemia 33, 64-74 (2019). https://doi.org/10.1038/s41375-018-0180-3) describe that targeted immunotherapy in acute myeloid leukemia (AML) is challenged by the lack of AML-specific target antigens and clonal heterogeneity, leading to unwanted on-target off-leukemia toxicity and risk of relapse from minor clones. They hypothesize that combinatorial targeting of AML cells can enhance therapeutic efficacy without increasing toxicity. To identify target antigen combinations specific for AML and leukemic stem cells, they generated a detailed protein expression profile based on flow cytometry of primary AML (*n* = 356) and normal bone marrow samples (*n* = 34), and a recently reported integrated normal tissue proteomic data set. They analyzed antigen expression levels of CD33, CD123, CLL1, TIM3, CD244 and CD7 on AML bulk and leukemic stem cells at initial diagnosis (*n* = 302) and relapse (*n* = 54). CD33, CD123, CLL1, TIM3 and CD244 were ubiquitously expressed on AML bulk cells at initial diagnosis and relapse, irrespective of genetic characteristics. For each analyzed target, they found additional expression in different populations of normal hematopoiesis. Analyzing the coexpression of our six targets in all dual combinations (*n* = 15), they found CD33/TIM3 and CLL1/TIM3 to be highly positive in AML compared with normal hematopoiesis and non-hematopoietic tissues. They propose that combinatorial targeting of CD33/TIM3 or CLL1/TIM3 may enhance therapeutic efficacy without aggravating toxicity in immunotherapy of AML

A targeted identification and elimination of AML stem cells remains an unmet need in leukemia treatment. There is also a continuing need in the art to identify agents with therapeutic potential for the treatment of proliferative disorders, such as AML and for improved specific diagnostic uses. The identification of these agents is urgently needed in order to allow the further development of therapies from the laboratory into the clinical practice.

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The above problem is solved in a first aspect of the present invention by a method for identifying an at least bispecific antigen binding molecule that binds specifically to mammalian leukemic stem cells (LSCs), the method comprising the steps of:
a) providing a first library of candidate antigen binding molecules specific for the protein CD 123 or immunologically recognizable fragments or derivatives thereof,
a') providing a second library of candidate antigen binding molecules specific for the protein CD200 or immunologically recognizable fragments or derivatives thereof,
b) combining a multitude of the candidate antigen binding molecules from the first and second library in order to produce a library of candidate molecules that are at least bispecific for CD123 and CD200,
c) providing a suitable screening system comprising the protein CD123 and/or CD200, or immunologically recognizable fragments or derivatives thereof,
d) contacting the library of b) with the screening system of c), and
e) identifying a binding molecule from the library of b) that specifically binds to the protein CD123 and CD200, or immunologically recognizable fragments or derivatives thereof.

Preferably, said LSCs are selected from LSC in acute myeloid leukemia (AML) or in chronic myeloid leukemia (CML).

The above problem is solved in a second aspect of the present invention by an at least bispecific antigen binding molecule that binds specifically to the proteins CD123 and CD200 or immunologically recognizable fragments or derivatives thereof of mammalian leukemic stem cells (LSCs), identified with a method according to the present invention.

In a third aspect thereof, the present invention relates to a pharmaceutical composition, comprising the at least bispecific antigen binding molecule according to the present invention, together with a pharmaceutically acceptable carrier and/or auxiliary agent.

The above problem is solved in a fourth aspect of the present invention by the at least bispecific antigen binding molecule according to the present invention or the pharmaceutical composition according to the present invention, for use in medicine, preferably for use in the prevention or treatment of cancer in a patient, such as leukemia, for example AML or CML, and in particular pediatric forms thereof, comprising administering a effective amount of the at least bispecific antigen binding molecule according to the present invention or the pharmaceutical composition according to the present invention to said patient.

The above problem is solved in a fifth aspect of the present invention by a method for preventing or treating a cancer, such as leukemia, for example AML or CML, and in particular pediatric forms thereof in a patient, comprising administering an effective amount of the at least bispecific antigen binding molecule according to the present invention or the pharmaceutical composition according to the present invention to said patient.

The above problem is solved in a sixth aspect of the present invention by a method for identifying mammalian leukemic stem cells (LSCs) in a biological sample obtained from a mammalian patient suspected to have leukemia, comprising
a) contacting the sample with the at least bispecific antigen binding molecule according to the present invention, and
b) detecting binding of the at least bispecific antigen binding molecule to cells in said sample,
wherein a binding, and in particular a specific binding of the at least bispecific antigen binding molecule to the cells identifies leukemic stem cells (LSCs) in said sample.

The above problem is solved in a seventh aspect of the present invention by a method for diagnosing leukemia in a mammalian patient suspected to have leukemia, comprising a method according to the present invention, and further concluding on leukemia in the mammalian patient based on the binding. Preferably, the leukemia is relapsing, malign, and/or metastasizing leukemia, AML, in particular pediatric form thereof, and CML.

As mentioned above, the present invention in a first aspect thereof relates to an in vitro method for identifying an at least bispecific antigen binding molecule that binds specifically to mammalian leukemic stem cells (LSCs), the method comprising the steps of:
a) providing a first library of candidate antigen binding molecules specific for the protein CD 123 or immunologically recognizable fragments or derivatives thereof,
a') providing a second library of candidate antigen binding molecules specific for the protein CD200 or immunologically recognizable fragments or derivatives thereof,
b) combining a multitude of the candidate antigen binding molecules from the first and second library in order to produce a library of candidate molecules that are at least bispecific for CD123 and CD200,
c) providing a suitable screening system comprising the protein CD123 and/or CD200, or immunologically recognizable fragments or derivatives thereof,
d) contacting the library of b) with the screening system of c), and
e) identifying a binding molecule from the library of b) that specifically binds to the protein CD123 and CD200, or immunologically recognizable fragments or derivatives thereof.

Preferred is a method according to the present invention, wherein said LSCs are selected from LSC in acute myeloid leukemia (AML) or in chronic myeloid leukemia (CML).

The present invention is generally based on the use of the target proteins cluster of differentiation ("CD") CD123 and CD200, since both markers are expressed together in leukemic stem cells, in particular AML-stem cells.

CD200 (also known as OX-2) is a membrane protein of the "Ig super family" (IgSF) and is expressed in several cell types (Barclay AN, Clark MJ, McCaughan GW. Neuronal/lymphoid membrane glycoprotein MRC OX-2 is a member of the immunoglobulin superfamily with a light-chain-like structure. Biochem Soc Symp. 1986;51: 149-157). The CD200 receptor (CD200R) can be found on cells of the hematopoietic lines (T, B and NK cells, as well as immune cells of the myeloid lineage). Binding of the receptors to CD200 inhibits these immune cells (Wright GJ, Cherwinski H, Foster-Cuevas M, Brooke G, Puklavec MJ, Bigler M, et al. Characterization of the CD200 receptor family in mice and humans and their interactions with CD200. J Immunol. 2003;171: 3034-3046). In many AML patients, CD200 is overexpressed on the malign cells and contributes to the "immunoevasion" of LSC (Herbrich S, Baggerly K, Alatrash G, Davis RE, Konopleva MY. CD200 Is a Stem Cell-Specific Immunosuppressive Target in AML. 2018;132: 2768-2768; Kawasaki BT, Mistree T, Hurt EM, Kalathur M, Farrar WL. Co-expression of the toleragenic glycoprotein, CD200, with markers for cancer stem cells. Biochem Biophys Res Commun. 2007;364: 778-782; Kawasaki BT, Farrar WL. Cancer stem cells, CD200 and immunoevasion. Trends Immunol. 2008;29: 464-468; Coles SJ, Wang ECY, Man S, Hills RK, Burnett AK, Tonks A, et al. CD200 expression suppresses natural killer cell function and directly inhibits patient anti-tumor response in acute myeloid leukemia. Leukemia. 2011;25: 792-799). A therapeutic approach with CD200 as a checkpoint-inhibitor was discussed as promising (Rastogi N, Baker S, Man S, Uger RA, Wong M, Coles SJ, et al. Use of an anti-CD200-blocking antibody improves immune responses to AML in vitro and in vivo. Br J Haematol. 2020. doi:10.1111/bjh.17125). A therapeutic antibody against CD200 (Samalizumab) is currently tested in patients with chronic lymphatic leukemia (CLL) and multiple myeloma (MM) in a clinical phase 1 study (Mahadevan D, Lanasa MC, Farber C, Pandey M, Whelden M, Faas SJ, et al. Phase I study of samalizumab in chronic lymphocytic leukemia and multiple myeloma: blockade of the immune checkpoint CD200. J Immunother Cancer. 2019;7: 227). Therefore, CD200 was chosen as a first target in the combinatorial approach according to the present invention.

CD123 (IL3RA) is a surface marker on hematopoetic precursor cells and is strongly overexpressed in AML-stem cell leukemia (SCL). This correlates witn an increased proliferation of AML-cells (see also below for the diagnostic methods). While this effect may be explained by a IL3-mediated signal transduction, the mechanisms of an increased resistance of CD123 positive AML cells against chemotherapy are not fully understood (Yan B, Chen Q, Shimada K, Tang M, Li H, Gurumurthy A, et al. Histone deacetylase inhibitor targets CD123/CD47-positive cells and reverse chemoresistance phenotype in acute myeloid leukemia. Leukemia. 2019;33: 931-944; Jordan CT, Upchurch D, Szilvassy SJ, Guzman ML, Howard DS, Pettigrew AL, et al. The interleukin-3 receptor alpha chain is a unique marker for human acute myelogenous leukemia stem cells. Leukemia. 2000;14: 1777-1784; Testa U, Pelosi E, Frankel A. CD 123 is a membrane biomarker and a therapeutic target in hematologic malignancies. Biomark Res. 2014;2: 4).

Several clinical trials are investigating the use of various CD123-targeting agents, including chimeric antigen receptor-modified T cells (expressing CD123, monoclonal antibodies, combined CD3-CD123 dual-affinity retargeting antibody therapy, recombinant fusion proteins, and CD123-engager T cells (El Achi H, Dupont E, Paul S, Khoury JD. CD123 as a Biomarker in Hematolymphoid Malignancies: Principles of Detection and Targeted Therapies. Cancers (Basel). 2020;12(11):3087. Published 2020 Oct 23. doi:10.3390/cancers12113087). Examples are CD123-neutralising antibodies, CD3×CD123-bispecific antibodies, dual-affinity retargeting (DART), and CAR-T-cells (Shi M, Su RJ, Parmar K-P, Chaudhry R, SunK, Rao J, et al. CD123: A Novel Biomarker for Diagnosis and Treatment of Leukemia. Cardiovasc Hematol Disord Drug Targets. 2019;19: 195-204; Search of: CD123. [cited 6 Dec 2021]. Available: https://clinicaltrials.gov/ct2/results?cond=aml&term=CD123&cntry=&state=&city=&di st=). The experimental antibody-drug conjugate SGN-CD123A targets CD123 as a possible treatment for AML

CD123 is disclosed as a second diagnostic target (Kandeel EZ, Madney Y, Eldin DN, Shafik NF. Overexpression of CD200 and CD123 is a major influential factor in the clinical course of pediatric acute myeloid leukemia. Exp Mol Pathol. 2021;118: 104597; Coustan-Smith E, Song G, Shurtleff S, Yeoh AE-J, Chng WJ, Chen SP, et al. Universal monitoring of minimal residual disease in acute myeloid leukemia. JCI Insight. 2018;3. doi:10.1172/jci.insight.98561).

Therefore, both target proteins do not only show a strong overexpression in AML-LSCs, but are furthermore important for a successful immunoevasion and an increased proliferation of the cells of the cancer. Since both target proteins represent not only markers but also contribute to the malignity of leukemia, such as AML, an otherwise common and highly undesired "target-loss" is unlikely.

In view of the above, the present inventors have examined an advantageous combined approach against the target proteins CD123 and CD200, and produced and tested new recombinant bispecific antigen binders, in particular a trifab-contorsbody.

Kandeel EZ, et al. (in: Overexpression of CD200 and CD123 is a major influential factor in the clinical course of pediatric acute myeloid leukemia. Exp Mol Pathol. 2021 Feb;118:104597. doi: 10.1016/j.yexmp.2020.104597. Epub 2020 Dec 23. PMID: 33358743) disclose that acute myeloid leukemia (AML) accounts for approximately 20% of all pediatric acute leukemias. The outcome of AML is still unsatisfactory. CD123 and CD200 were demonstrated to play important roles in hematological malignancies. CD200 and CD123 both had a negative influence on clinical presentation and treatment outcome, which remarkably worsened when both were concomitantly overexpressed. CD200 and CD123 can therefore be used as markers of minimal residual disease in AML. They also speculate about the use as therapeutic targets, but there is no further information regarding this.

In the context of the present invention, the term "CD123" generally refers to the interleukin-3 receptor (CD123) protein, subunit alpha, a molecule found on cells which helps transmit the signal of interleukin-3, a soluble cytokine important in the immune system. The term shall encompass the human version of this protein, but also its homologs, in particular in mouse or rat. Information on CD123 can be derived from the human gene names webpage (https://www.genenames.org/) which the accession number HGNC:6012. The CD123 protein is accessible on the UniProt webpage under the accession number P26951, and is further, at least the human version, shown in SEQ ID NO: 1 (isoform 1). All other isoforms of the protein shall also be encompassed by the present invention.

In the context of the present invention, the term "CD200" generally refers to the CD200 molecule protein, also referred to as OX-2 membrane glycoprotein. The term shall encompass the human version of this protein, but also its homologs, in particular in mouse or rat. Information on CD200 can be derived from the human gene names webpage (https://www.genenames.org/) which the accession number HGNC:7203. The CD200 protein is accessible on the UniProt webpage under the accession number P41217 (isoform 1), and is further, at least the human version, shown in SEQ ID NO: 2. All other isoforms of the protein shall also be encompassed by the present invention.

Fragments or derivatives or variants of CD200 and/or CD123 can be readily tested for the ability to be able to bind antigen-binders according to the present invention. Hence, the present invention also discloses a method for determining whether a protein derivative, variant and/or fragment of CD200 and/or CD123 maintains the ability to interact (bind) to bind antigen-binders according to the present invention, and thus constitutes an immunologically recognizable fragment. The method comprises a step of contacting the candidate derivative, variant and/or fragment of CD200 and/or CD123 with the antigen binding molecule of the invnetion, and detecting their interaction, i.e., binding. The above method for testing protein variants/derivatives and/or fragments may in some embodiments form part of the afore described method for the identification, preferably if the method encompasses the use of any protein derivative, variant and/or fragment of CD123 and/or CD200. These fragments may also be included in the screening system as used in the context of the present invention.

Since the CD123 protein is composed of three extracellular domains (287 amino acids), a single-pass transmembrane domain (30 amino acids), and a short intracellular region (53 amino acids), a preferred fragment for use in the context of the present invention is the extracellular part, which may be used as a soluble protein, which furthermore can be labeled and/or recombinantly produced.

CD200 is composed of two extracellular (one variable and one constant) immunoglobulin-like domains, a single transmembrane region, and a cytoplasmic tail. Thus, a preferred fragment for use in the context of the present invention is the extracellular part, which may be used as a soluble protein, which furthermore can be labeled and/or recombinantly produced.

In context of the invention, the terms "CD123" and/or "CD200" shall also include other proteins, their variants or fragments if they have an amino acid with a sequence identity of at least 50, 60, 70, 80, 85, 86, 87, 88, 89, 90,91, 92, 93, 94, 95, 96, 97, 98, 99 or 100% compared to the amino acid sequence human CD123 or CD200, respectively, e.g. to SEQ ID NO: 1 or 2, respectively, as disclosed herein. Fragments of CD123 or CD200 or their variants, shall preferably have a length of at least 30, 40, 50, 80, 100, 150, 200, 300 or more amino acids. A variant may comprise conservative amino acid changes, as known to the person of skill.

As used herein, the terms "identical" or percent "identity", when used herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences -, when compared and aligned for maximum correspondence over the comparison window or designated region) as measured using a sequence comparison algorithms, or by manual alignment and visual inspection (see, e.g., NCBI web site). In particular for amino acid identity, the use of BLASTP 2.2.28+ with the following parameters is encompassed: Matrix: BLOSUM62; Gap Penalties: Existence: 11, Extension: 1; Neighboring words threshold: 11; Window for multiple hits: 40.

In the method according to the present invention, any suitable library of candidate antigen binding molecules that allows for a selection of domains involved in antigen recognition to be used as binding moieties in the design of the bispecific diagnostics and therapeutics can be used. Preferably, the library is selected from an antibody library or a phage display library, more preferably a human-based or humanized-based library. Examples for the domains involved in antigen recognition include domain antibodies (heavy chain-only variable domain (V_{HH})) and single-chain Fv fragments (scFvs), antigen-binding fragments (Fabs), single-chain Fab fragments (scFabs) and, more recently, single-chain Fc fragments (scFcs), and preferably comprises single-chain variable fragments (scFvs) or stabilized form thereof (e.g., a disulfide stabilized scFv), Fab fragments, Fab' fragments, F(ab') fragments, F(ab')₂ fragments, scFabs, and single domain antibody fragments, such as heavy chain-only variable domain (VHH), single-domain antibodies (sdAb), or nanobodies. Furthermore, TCR recognition domains may be included, i.e., a construct including the essential CDRs of a TCR that form the loops protruding from the core Vα or Vβ domain and mediate the specific binding.

Once suitable binding domains/molecules are identified (e.g., by additional antigen binding tests as disclosed herein and/or sequencing of the respective nucleic acid molecules), these are combined into suitable bispecifc constructs to be used in the context of the present invention. The present invention relates to a method according to the present invention, wherein the combining comprises a covalent or non-covalent fusion or combination of the candidate antigen binding molecules from the first and second library. The combining may include additional steps of cloning or otherwise inserting (e.g. synthesizing a polypeptide) the binding domains/molecules into suitable scaffold structures. Respective methods are described in the literature and well known to the person of skill.

The at least bispecific binder for CD123 and CD200 can be of any suitable format that allows for the binding, preferably simultaneous, binding of CD123 and CD200 in diagnostic assays, screening assays and/or therapeutic applications as disclosed herein.

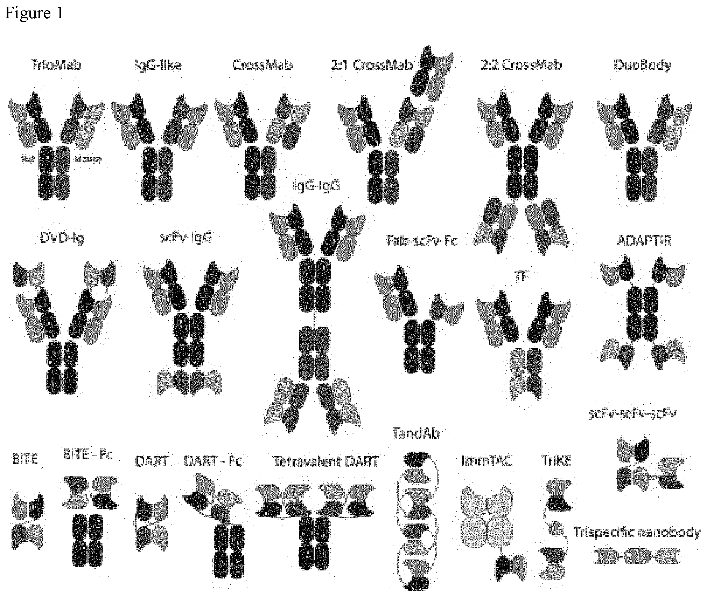

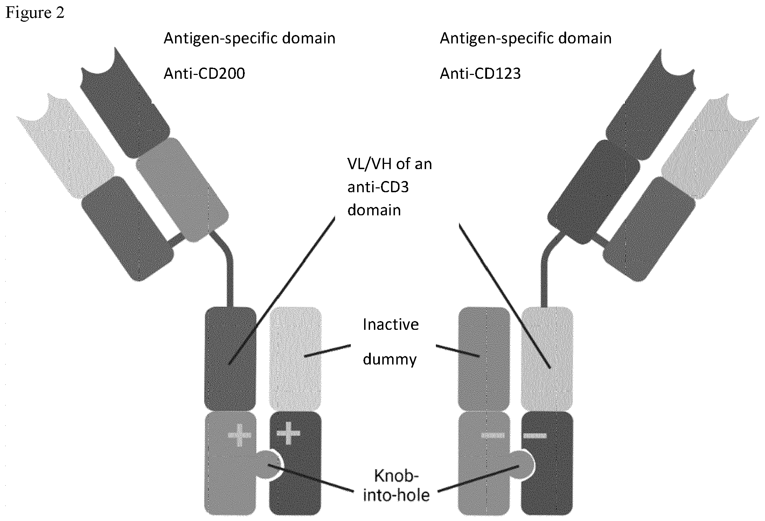

Preferred is a method according to the present invention, wherein the library of candidate molecules that are at least bispecific binder for CD123 and CD200, i.e. the anticipated "final product" comprises at least one bispecific format selected from the group consisting of TrioMab, IgG-like, CrossMab, 2:1 CrossMab, 2:2 CrossMab, DuoBody, DVD-Ig, scFv-IgG, IgG-IgG, Fab-scFv-Fc, TF, ADAPTIR, BITE, BITE-Fc, DART, DART-FC, Tetravalent DART, TandAb, ImmTAC, TriKE, scFv-scFv-scFv, Trispecific nanobody, a diabody, triabody, tetrabody or higher order multimer, and a trifab-contorsbody (see Figures 1 and 2, and Suurs FV, Lub-de Hooge MN, de Vries EGE, de Groot DJA. A review of bispecific antibodies and antibody constructs in oncology and clinical challenges. Pharmacol Ther. 2019 Sep;201:103-119. doi: 10.1016/j.pharmthera.2019.04.006. Epub 2019 Apr 24. PMID: 31028837).

More preferred is a method according to the present invention, wherein the candidate molecules of the library further comprise at least one third antigen binding domain, for example a CD3 binding domain. This allows for additional functions, particularly in immunotherapy or diagnostic applications, but also could include a binder for the purpose of binding to a matrix or other protein or protein complex. Further preferred examples are specific binders to CD33, TIM3 and/or CLL1, or additional T-cell specific antigens, such as CD4, CD8, or CD28.

More preferred is a method according to the present invention, wherein the candidate molecules of the library as above further comprise Fc-fragments or mutated or inactivated derivatives/versions thereof, such as, for example, Fc-fragments missing cysteine residues and/or cysteine-bridges and/or including "knob" or "hole" structures.

A particularly preferred construct/molecule as produced constitues an at least bispecific binder for CD123 and CD200 in the form of a trifab-contorsbody (see, for example Figure 2 and EP3704146). The trifab molecule is a T-cell-activating antibody format that comprises two complementary halves and is specifically directed at antigen combinations. Each half of the molecule consists of a light and a heavy antibody chain. In addition, the construct includes a so-called "dummy" chain. Like for a regular IgG antibody, the light chain and the heavy chain with the "variable light" (VL) and "variable heavy (VH) chain domain" constitue the antigen specific binding domain. The Fc-fragment (fragment crystallisable) has been modified as follows; no cysteine bonds with another heavy chain, the CD2 domain was replaced by the VL of an anti-CD3-antibody in the first half, and by the VH of an anti-CD3-antibody in the second half (Mayer K, Baumann A-L, Grote M,_Seeber S, Kettenberger H, Breuer S, et al. TriFabs--Trivalent IgG-Shaped Bispecific Antibody Derivatives: Design, Generation, Characterization and Application for Targeted Payload Delivery. Int J Mol Sci. 2015;16: 27497-27507). Furthermore, the knob-into-hole system was introduced that avoids a dimerisation of two identical halves (see Ridgway JB, Presta LG, Carter P. "Knobs-into-holes" engineering of antibody CH3 domains for heavy chain heterodimerization. Protein Eng. 1996;9: 617-621). Therefor, the halves may be designated as "knob" or "hole", respectively. Because of the modifcations, the Fc-fragment is rendered inactive. The "dummy" chain is complemetary to the respective Fc-fragment but can not generate an active anti-CD3 domain.

If only one half of the construct binds to a cell where only one target is expressed, i.e., either CD123 or CD200, T cells will not be activated. Only if both target structures are recognized on the surface of a cell, an exchange of the chains takes place that is thermodynamically promoted by the charges in the Fc fragment. A reshuffling leads to a removal of both "dummy chains", while a functional anti-CD3 domain is formed, thus only conditionally ("only if') activating the T-cells. The desired cytotoxic activity is therefore limited and localized only to those cells that express CD200 and CD123.

LSCs can be identified from any mammal that has LSCs expressing CD200 and CD123 or homologs thereof. Preferred is the method according to the present invention, wherein the mammal is selected from a mouse, monkey, dog, cat, rat, or human, and preferably a human child.

The method according to the present invention involves a suitable system for identifying and/or screening for a binding of the antigen binding molecules of the libraries. The system to be used in accordance with the present invention can be and comprise any suitable means for identifying, and in particular specifically identifying, a binding of the antigen binding molecules to either CD123 or CD200 or antigenic fragments thereof. Preferably, such a system comprises a cell, such as a bacterial or yeast cell or human cell, expressing, and in particular recombinantly expressing, CD123 and CD200 or antigenic fragments thereof. The system may be a cell-free system, either in solution or involving the binders and/or the CD123 or CD200 or antigenic fragments thereof bound or conjugated to a matrix. The system furthermore may include suitable reporter moieties or labels. A preferred antigenic fragment for use in the context of the present invention is the extracellular part of CD123 or CD200 (see also above), which may be used as a soluble protein or attached to a matrix, and furthermore can be labeled and/or recombinantly produced. Preferred is the method according to the present invention, wherein the candidate molecules of the library further comprise at least one marker or label, such as a toxin, enzyme, or fluorophore.

Furthermore, the present invention in a second aspect thereof relates to an at least bispecific antigen binding molecule that binds specifically to the proteins CD123 and CD200 or immunologically recognizable fragments or derivatives thereof of mammalian leukemic stem cells (LSCs) that were identified with a method according to the present invention. The at least bispecific binder for CD123 and CD200 can be of any suitable format that allows for the binding, preferably simultaneous, binding of CD123 and CD200 in diagnostic assays, screening assays and/or therapeutic applications as disclosed herein, and preferably comprises at least one bispecific format selected from the group consisting of TrioMab, IgG-like, CrossMab, 2:1 CrossMab, 2:2 CrossMab, DuoBody, DVD-Ig, scFv-IgG, IgG-IgG, Fab-scFv-Fc, TF, ADAPTIR, BITE, BITE-Fc, DART, DART-FC, Tetravalent DART, TandAb, ImmTAC, TriKE, scFv-scFv-scFv, Trispecific nanobody, a diabody, triabody, tetrabody or higher order multimer, and a trifab-contorsbody (see Figures 1 and 2, and Suurs FV, Lub-de Hooge MN, de Vries EGE, de Groot DJA. A review of bispecific antibodies and antibody constructs in oncology and clinical challenges. Pharmacol Ther. 2019 Sep;201:103-119. doi: 10.1016/j.pharmthera.2019.04.006. Epub 2019 Apr 24. PMID: 31028837). Furthermore, TCR recognition domains may be included, i.e. a construct including the essential CDRs of a TCR that form the loops protruding from the core Vβ or Vβ domain and mediate the specific binding.

Preferred is the at least bispecific antigen binding molecule according to the present invention, wherein the antigen binding domains thereof are bound covalently or non-covalently.

More preferred is the at least bispecific antigen binding molecule according to the present invention, wherein the candidate molecules of the library further comprise at least one third antigen binding domain, for example a CD3 binding domain. This allows for additional functions, particularly in immunotherapy or diagnostic applications, but also can include a binder for the purpose of binding to a matrix or other protein or protein complex. Further preferred examples are specific binders to CD33, TIM3 and/or CLL1, or additional T-cell specific antigens, such as CD4, CD8, or CD28.

A particularly preferred construct/molecule as produced constitues an at least bispecific binder for CD123 and CD200 in the form of a trifab-contorsbody as mentioned above. Another particularly preferred construct/molecule as produced constitues an at least bispecific binder for CD123 and CD200 that simultaneously binds CD123 and CD200 on the surface of an LSC.

Another particularly preferred embodiment of the construct/molecule as produced according to the invention constitues an at least bispecific binder for CD123 and CD200 upon binding inhibits the biological function of CD123 CD200 or both, e.g. the signaling thereof, in an cell, such as an LSC.

More preferred is the at least bispecific antigen binding molecule according to the present invention, wherein the molecules of the library as above further comprise Fc-fragments or mutated or inactivated derivatives/versions thereof, such as, for example, Fc-fragments missing cysteine residues and/or cysteine-bridges and/or including "knob" or "hole" structures.

Preferred is the at least bispecific antigen binding molecule according to the present invention, wherein said LSCs are selected from LSC in acute myeloid leukemia (AML) or in chronic myeloid leukaemia (CML).

Preferred is the at least bispecific antigen binding molecule according to the present invention, wherein the candidate molecules of the library further comprise at least one marker or label, such as a toxin, enzyme, or fluorophore.

A preferred example is the at least bispecific antigen binding molecule according to the present invention, wherein the amino acid sequence of the CD123 binding region is as disclosed in EP2778175A1 (incorporated by reference in its entirety) or specifically binding sequences that are at least 95% identical to the sequence as disclosed, and wherein the amino acid sequence of the CD200 binding region is according to EP2178561A1 (incorporated by reference in its entirety) or specifically binding sequences that are at least 95% identical to the sequence as disclosed.

Another preferred example is the at least bispecific antigen binding molecule according to the present invention, wherein the amino acid sequence of the light chain variable region (V_{L}) for binding to CD123 comprises an amino acid sequence according to SEQ ID NO: 3, the amino acid sequence of the heavy chain variable region (V_{H}) for binding to CD123 comprises an amino acid sequence according to SEQ ID NO: 4, and optionally the heavy chain constant region comprises amino acid substitutions S239D and I332E according to the EU numbering system of Kabat, or specifically binding sequences that are at least 95% identical to the sequences.

Yet another preferred example is the at least bispecific antigen binding molecule according to the present invention, wherein the amino acid sequence of the light chain for binding to CD123 comprises an amino acid sequence according to SEQ ID NO: 5, the amino acid sequence of the heavy chain for binding to CD123 comprises an amino acid sequence according to SEQ ID NO: 6 or specifically binding sequences that are at least 95% identical to the sequences.

Yet another preferred example is the at least bispecific antigen binding molecule according to the present invention, wherein the amino acid sequence of the CD200-binding fragment thereof, contains one of the following paired sets of CDRs:
(i) a HCDR1 comprising the amino acid sequence: GFNIKDYYMH (SEQ ID NO:7); a HCDR2 comprising the amino acid sequence: WIDPENGDTKYAPKFQG (SEQ ID NO:8); a HCDR3 comprising the amino acid sequence: KNYYVSNYNFFDV (SEQ ID NO:9); a LCDR1 comprising the amino acid sequence: SASSSVRYMY (SEQ ID NO:10); a LCDR2 comprising the amino acid sequence: DTSKLAS (SEQ ID NO:11); and a LCDR3 comprising the amino acid sequence: FQGSGYPLT (SEQ ID NO:12);
(ii) a HCDR1 comprising the amino acid sequence: GFNIKDYYIH (SEQ ID NO:13); a HCDR2 comprising the amino acid sequence: WIDPEIGATKYVPKFQG (SEQ ID NO:14); a HCDR3 comprising the amino acid sequence: LYGNYDRYYAMDY (SEQ ID NO:15); a LCDR1 comprising the amino acid sequence: KASQNVRTAVA (SEQ ID NO:16); a LCDR2 comprising the amino acid sequence: LASNRHT (SEQ ID NO:17); and a LCDR3 comprising the amino acid sequence: LQHWNYPLT (SEQ ID NO:18);
(iii) a HCDR1 comprising the amino acid sequence: GYSFTDYIIL (SEQ ID NO:19); a HCDR2 comprising the amino acid sequence: HIDPYYGSSNYNLKFKG (SEQ ID NO:20); a HCDR3 comprising the amino acid sequence: SKRDYFDY (SEQ ID NO:21); a LCDR1 comprising the amino acid sequence: KASQDINSYLS (SEQ ID NO:22); a LCDR2 comprising the amino acid sequence: RANRLVD (SEQ ID NO:23); and a LCDR3 comprising the amino acid sequence: LQYDEFPYT (SEQ ID NO:24);
(iv) a HCDR1 comprising the amino acid sequence: GYTFTEYTMH (SEQ ID NO:25); a HCDR2 comprising the amino acid sequence: GVNPNNGGALYNQKFKG (SEQ ID NO:26); a HCDR3 comprising the amino acid sequence: RSNYRYDDAMDY (SEQ ID NO:27); a LCDR1 comprising the amino acid sequence: KSSQSLLDIDEKTYLN (SEQ ID NO:28); a LCDR2 comprising the amino acid sequence: LVSKLDS (SEQ ID NO:29); and a LCDR3 comprising the amino acid sequence: WQGTHFPQT (SEQ ID NO:30); or
(v) a HCDR1 comprising the amino acid sequence: AFNIKDHYMH (SEQ ID NO:31); a HCDR2 comprising the amino acid sequence: WIDPESGDTEYAPKFQG (SEQ ID NO:32); a HCDR3 comprising the amino acid sequence: FNGYQALDQ (SEQ ID NO:33); a LCDR1 comprising the amino acid sequence: TASSSVSSSYLH (SEQ ID NO:34); a LCDR2 comprising the amino acid sequence: STSNLAS (SEQ ID NO:35); and a LCDR3 comprising the amino acid sequence: RQYHRSPPIFT (SEQ ID NO:36), or specifically binding sequences that are at least 95% identical to the above sequences, preferably that are at least 96%, 97%, 98% or 99% identical to the above sequences.

In the above, HCDR designates the heavy chain complement determining region; and LCDR designates the light chain complement determining region of the antigen binding molecule binding domain.

More preferred is the at least bispecific antigen binding molecule according to the present invention, comprising a combination of the sequences as disclosed above, i.e. sequences selected from SEQ ID Nos: 3 to 6, combined with sequences selected from SEQ ID Nos: 7 to 12, 13 to 18, 19 to 24, 25 to 30, and 31 to 36, or specifically binding sequences that are at least 95% identical to the sequences.

Preferred is the at least bispecific antigen binding molecule according to the present invention, wherein the sequences as used, for example the binding sequences, are selected from murine, chimeric, humanized, and fully human sequences.

Yet another aspect of the present invention releates to a method for the production of a pharmaceutical composition, the method comprising formulating the at least bispecific antigen binding molecule according to the present invention, e.g. as identified, as a pharmaceutical composition together with a pharmaceutically acceptable carrier and/or excipient.

Yet another aspect of the present invention releates to a pharmaceutical composition, comprising the at least bispecific antigen binding molecule according to the present invention, together with a pharmaceutically acceptable carrier and/or auxiliary agent. Preferably, the pharmaceutical composition is suitable for the immunotherapy of leukemia, in particular AML and pediatric forms thereof.

As used herein the language "pharmaceutically acceptable" carrier, stabilizer or excipient is intended to include any and all solvents, solubilizers, fillers, stabilizers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical) and transmucosal administration.

Pharmaceutical compositions as used may comprise a pharmaceutically acceptable carrier. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for respective compounds, for example topical, and will readily be able to choose suitable pharmaceutically acceptable carriers or excipients, depending, e.g., on the formulation and administration route of the pharmaceutical composition.

Yet another aspect of the present invention releates to the at least bispecific antigen binding molecule according to the present invention or the pharmaceutical composition according to the present invention, for use in medicine. Preferred is the at least bispecific antigen binding molecule according to the present invention or the pharmaceutical composition according to the present invention for use in the prevention or treatment of cancer in a patient, such as leukemia, for example AML or CML, and in particular pediatric forms thereof, comprising administering a effective amount of the at least bispecific antigen binding molecule according to the present invention or the pharmaceutical composition according to the present invention to a patient in need thereof, wherein the patient is selected from a mouse, monkey, dog, cat, rat, or human, and preferably a human child.

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms.

Preferred is the at least bispecific antigen binding molecule or the pharmaceutical composition for use according to the present invention, wherein said cancer prevention or treatment comprises immunotherapy and/or includes the avoidance of target loss and/or metastasis of said cancer.

Yet another aspect of the present invention releates to a method for preventing or treating a cancer, such as leukemia, for example AML or CML, and in particular pediatric forms thereof in a patient, comprising administering an effective amount of the at least bispecific antigen binding molecule according to the present invention or the pharmaceutical composition according to the present invention to said patient. As above, the patient may be selected from a leukemia patient that is selected from a mouse, monkey, dog, cat, rat, or human, and preferably a human child.

Preferred is the method according to the present invention, wherein said cancer prevention or treatment comprises immunotherapy and/or includes the avoidance of target loss and/or metastasis of said cancer as described herein.

Methods for preventing or treating a disease using bispecific antigen binders are known to the person of skill, and are disclosed in the art, for example in Ma et al. (Bispecific Antibodies: From Research to Clinical Application. Front Immunol. 2021 May 5;12:626616. doi: 10.3389/fimmu.2021.626616. PMID: 34025638; PMCID: PMC8131538).

In another important aspect of the present invention, the above object is solved by a method for identifying mammalian leukemic stem cells (LSCs) in a biological sample obtained from a mammalian patient suspected to have leukemia, comprising
a) contacting the sample with the at least bispecific antigen binding molecule according to the present invention, and
b) detecting binding of the at least bispecific antigen binding molecule to cells in said sample,
wherein a binding, and in particular a specific binding and/or simultaneous binding of the at least bispecific antigen binding molecule to the CD123 and CD200 on the cells identifies/is indicative for leukemic stem cells (LSCs) in said sample.

In this aspect, the at least bispecific antigen binding molecule according to the present invention is used to identify leukemic stem cells (LSCs) in a sample. The method can be performed in vitro, in vivo or ex vivo. The sample can be any suitable sample including or suspected to include LSCs, such as a sample comprising blood cells or a sample of hematopoietic organs, such as bone marrow (BM), lymph nodes and spleen.

Preferred is the method for identifying mammalian leukemic stem cells (LSCs) in a biological sample according to the present invention, wherein the at least bispecific antigen binding molecule comprises at least one detectable marker, enzyme or label, such as a toxin, enzyme, or fluorophore. These markers, enzymes or labels allow for a detection of the antigen binding molecule on the surface of the LSCs, i.e. when bound to CD123 and CD200.

Preferred is the method for identifying mammalian leukemic stem cells (LSCs) in a biological sample according to the present invention, wherein the at least bispecific antigen binding molecule binds CD123 and CD200 simultaneously. The step of identifying may further comprise the identification of additional suitable markers of LSCs as described above.

A particular preferred aspect of the method according to the invention involves the trifab construct as described above, where a CD3 activity (e.g. T-cell activation) can be used to identify a simultaneous binding to CD123 and CD200 of the LSCs.

In another important aspect of the present invention, the above object is solved by a method for diagnosing (LSC-related) leukemia in a mammalian patient suspected to have leukemia, comprising a method according to the present invention, and further concluding on leukemia in the mammalian patient based on the binding. Preferably, the method according to the present invention further comprises a diagnosis whether the leukemia is relapsing, malign, and/or metastasizing leukemia, AML, in particular pediatric form thereof, and CML. The diagnosis can include a quatification of the LSCs in a sample derived from a patient. Since CD200 and CD123 both have a negative influence on clinical presentation and treatment outcome, which remarkably worsens when both are concomitantly overexpressed, CD200 and CD123 can therefore be used as markers of minimal residual disease in AML or the malign state of the disease and the risk for metastases, and a prognosis for the disease, where a higher quantity of CD200 and CD123 carrying cells as detected in the patient predicts a worse outcome. The markers CD200 and CD123 can also be used as markers for the sternness of the LSC. The markers CD200 and CD123 can also be used for stratifying AML patients.

The above methods can also be performed in the context of monitoring the prevention or treatment of a cancer, such as leukemia, for example AML or CML, and in particular pediatric forms thereof in a patient, comprising the methods for identifying or diagnosing as above on a sample derived from a patient during the course of a treatment, and comparing the results with a control sample, in particular a sample from the patient taken at the time before the prevention or treatment of a cancer, such as leukemia, for example AML or CML, and in particular pediatric forms thereof in a patient. Preferred is a prevention or treatment that comprises administering an effective amount of the at least bispecific antigen binding molecule according to the present invention or the pharmaceutical composition according to the present invention to said patient.

In yet another aspect of the present invention, the at least bispecific antigen binding molecule according to the present invention is used to purify and/or isolate LSCs expressing CD123 and CD200. Preferably, the at least bispecific antigen binding molecule binds CD123 and CD200 simultaneously, and is attached to a solid matix, such as a column material, beads and/or a membrane. After the LSCs in sample are contacted with the at least bispecific antigen binding molecule according to the present invention, the LSCs via CD123 and CD200 attach to the at least bispecific antigen binding molecule and may be isolated after washing and elution steps as a fraction enriched in LSCs, which may be used further in the establishment of cell lines or research.

The present inventors have designed and studied combined antigen binders for the target structures CD123 and CD200, and thus developed a new method for the elimination of leukemic stem cells. The CD123/CD200-dependent and advantageously localized activation of T-cells is an improved strategy for immunotherapy of leukemia, such as pediatric AML.

The present invention preferably relates to the following items:
Item 1. A method or in vitro method for identifying an at least bispecific antigen binding molecule that binds specifically to mammalian leukemic stem cells (LSCs), the method comprising the steps of:
   a) providing a first library of candidate antigen binding molecules specific for the protein CD123 or immunologically recognizable fragments or derivatives thereof,
   a') providing a second library of candidate antigen binding molecules specific for the protein CD200 or immunologically recognizable fragments or derivatives thereof,
   b) combining a multitude of the candidate antigen binding molecules from the first and second library in order to produce a library of candidate molecules that are at least bispecific for CD123 and CD200,
   c) providing a suitable screening system comprising the protein CD123 and/or CD200, or immunologically recognizable fragments or derivatives thereof,
   d) contacting the library of b) with the screening system of c), and
   e) identifying a binding molecule from the library of b) that specifically binds to the protein CD123 and CD200, or immunologically recognizable fragments or derivatives thereof.
Item 2. The method according to Item 1, wherein said LSCs are selected from LSC in acute myeloid leukemia (AML) or in chronic myeloid leukemia (CML).
Item 3. The method according to Item 1 or 2, wherein the library of candidate antigen binding molecules is selected from an antibody library or a phage display library, preferably comprising single-chain variable fragments (scFvs) or stabilized form thereof (e.g., a disulfide stabilized scFv), Fab fragments, Fab' fragments, F(ab') fragments, F(ab')₂ fragments, scFabs, and single domain antibody fragments, such as heavy chain-only variable domain (VHH), Single-domain antibodies (sdAb), or nanobodies.
Item 4. The method according to any one of Items 1 to 3, wherein the combining comprises a covalent or non-covalent fusion or combination of the candidate antigen binding molecules from the first and second library.
Item 5. The method according to any one of Items 1 to 4, wherein the library of candidate molecules that are at least bispecific binder for CD123 and CD200 comprises at least one bispecific format selected from the group consisting of TrioMab, IgG-like, CrossMab, 2:1 CrossMab, 2:2 CrossMab, DuoBody, DVD-Ig, scFv-IgG, IgG-IgG, Fab-scFv-Fc, TF, ADAPTIR, BITE, BITE-Fc, DART, DART-FC, Tetravalent DART, TandAb, ImmTAC, TriKE, scFv-scFv-scFv, Trispecific nanobody, a diabody, triabody, tetrabody or higher order multimer, and a trifab-contorsbody.
Item 6. The method according to any one of Items 1 to 5, wherein the candidate molecules of the library further comprise at least one third antigen binding domain, for example a CD3 binding domain.
Item 7. The method according to any one of Items 1 to 6, wherein the candidate molecules of the library further comprise Fc-fragments or mutated or inactivated versions thereof, such as Fc-fragments missing cysteine residues and/or cysteine-bridges, or including knob or hole structures.
Item 8. The method according to any one of Items 1 to 6, wherein the mammal is selected from a mouse, monkey, dog, cat, rat, or human, and preferably a human child.
Item 9. The method according to any one of Items 1 to 7, wherein the suitable screening system comprises a cell expressing, and in particular recombinantly expressing, CD123 and CD200 or antigenic fragments thereof, or is a cell-free system.
Item 10. The method according to any one of Items 1 to 9, wherein the candidate molecules of the library further comprise at least one marker or label.
Item 11. An at least bispecific antigen binding molecule that binds specifically to the proteins CD123 and CD200 or immunologically recognizable fragments or derivatives thereof of mammalian leukemic stem cells (LSCs), identified with a method according to any one of Items 1 to 10.
Item 12. The at least bispecific antigen binding molecule according to Item 11, wherein said LSCs are selected from LSC in acute myeloid leukemia (AML) or in chronic myeloid leukaemia (CML).
Item 13. The at least bispecific antigen binding molecule according to Item 11 or 12, wherein the antigen binding domains thereof are bound covalently or non-covalently.
Item 14. The at least bispecific antigen binding molecule according to any one of Items 11 to 13, selected from an at least one bispecific format selected from the group consisting of TrioMab, IgG-like, CrossMab, 2:1 CrossMab, 2:2 CrossMab, DuoBody, DVD-Ig, scFv-IgG, IgG-IgG, Fab-scFv-Fc, TF, ADAPTIR, BITE, BITE-Fc, DART, DART-FC, Tetravalent DART, TandAb, ImmTAC, TriKE, scFv-scFv-scFv, Trispecific nanobody, a diabody, triabody, tetrabody or higher order multimer, and a trifab-contorsbody.
Item 15. The at least bispecific antigen binding molecule according to any one of Items 11 to 14, wherein the candidate molecules of the library further comprise at least one third antigen binding domain, for example a CD3 binding domain.
Item 16. The at least bispecific antigen binding molecule according to any one of Items 11 to 15, wherein the molecule further comprises Fc-fragments or mutated or inactivated versions thereof, such as Fc-fragments missing cysteine residues and/or cysteine-bridges, or including knob or hole structures.
Item 17. The at least bispecific antigen binding molecule according to any one of Items 11 to 16, wherein the candidate molecules of the library further comprise at least one marker or label.
Item 18. The at least bispecific antigen binding molecule according to any one of Items 11 to 17, wherein the amino acid sequence of the CD123 binding region is according to EP2778175A1 or specifically binding sequences that are at least 95% identical to the sequence, and wherein the amino acid sequence of the CD200 binding region is according to EP2178561A1 or specifically binding sequences that are at least 95% identical to the sequence.
Item 19. The at least bispecific antigen binding molecule according to any one of Items 11 to 18, which is murine, chimeric, humanized, or fully human.
Item 20. A pharmaceutical composition, comprising the at least bispecific antigen binding molecule according to any one of Items 11 to 19, together with a pharmaceutically acceptable carrier and/or auxiliary agent.
Item 21. The at least bispecific antigen binding molecule according to any one of Items 11 to 19 or the pharmaceutical composition according to Item 20, for use in medicine, preferably for use in the prevention or treatment of cancer in a patient, such as leukemia, for example AML or CML, and in particular pediatric forms thereof, comprising administering a effective amount of the at least bispecific antigen binding molecule according to any one of Items 11 to 18 or the pharmaceutical composition according to Item 19 to said patient.
Item 22. The at least bispecific antigen binding molecule or the pharmaceutical composition for use according to Item 21, wherein the patient is selected from a mouse, monkey, dog, cat, rat, or human, and preferably a human child.
Item 23. The at least bispecific antigen binding molecule or the pharmaceutical composition for use according to Item 21 or 22, wherein said cancer prevention or treatment comprises immunotherapy and/or includes the avoidance of target loss and/or metastasis of said cancer.
Item 24. A method for preventing or treating a cancer, such as leukemia, for example AML or CML, and in particular pediatric forms thereof in a patient, comprising administering an effective amount of the at least bispecific antigen binding molecule according to any one of Items 11 to 18 or the pharmaceutical composition according to Item 19 to said patient.
Item 25. The method according to Item 24, wherein the patient is selected from a mouse, monkey, dog, cat, rat, or human, and preferably a human child.
Item 26. The method according to Item 25 or 26, wherein said cancer prevention or treatment comprises immunotherapy and/or includes the avoidance of target loss and/or metastasis of said cancer.
Item 27. A method for identifying mammalian leukemic stem cells (LSCs) in a biological sample obtained from a mammalian patient suspected to have leukemia, comprising
   a) contacting the sample with the at least bispecific antigen binding molecule according to any one of Items 11 to 17, and
   b) detecting binding of the at least bispecific antigen binding molecule to cells in said sample,
   wherein a binding, and in particular a specific binding of the at least bispecific antigen binding molecule to the cells identifies leukemic stem cells (LSCs) in said sample.
Item 28. The method for identifying mammalian leukemic stem cells (LSCs) in a biological sample according to Item 27, wherein the at least bispecific antigen binding molecule comprises at least one detectable marker, enzyme or label.
Item 29. The method for identifying mammalian leukemic stem cells (LSCs) in a biological sample according to Item 27 or 28, wherein the at least bispecific antigen binding molecule binds CD123 and CD200 simultaneously.
Item 30. A method for diagnosing leukemia in a mammalian patient suspected to have leukemia, comprising a method according to any one of Items 27 to 29, and further concluding on leukemia in the mammalian patient based on the binding.
Item 31. The method according to Item 30, wherein said leukemia is relapsing, malign, and/or metastasizing leukemia, AML, in particular pediatric form thereof, and CML.
Item 32. Use of the at least bispecific antigen binding molecule according to any one of Items 11 to 18 to purify and/or isolate LSCs.

The present invention will now be further described in the following examples with reference to the accompanying figures and sequences, nevertheless, without being limited thereto. For the purposes of the present invention, all references as cited herein are incorporated by reference in their entireties.:
Figure 1: Schematic overview of the structure of bsAb constructs currently being evaluated in clinical trials. The IgG antibody construct consists of Fab and Fc regions. The binding part of the Fab region is called the single chain variable fragment (scFv). The antibody exists of two heavy chains (V_{H} and C_{H}) and two light chains (V_{L} and C_{L}). These chains can be subdivided by variable (V_{H} and V_{L}) and constant domains (C_{H} and C_{L}). Taken from Suurs FV, et al., 2019.
Figure 2: Structure of a trifab-contorsbody according to an embodiment of the invention. Upon simultaneous binding of the constructs to CD200 and CD123, the inactive dummy chains are replaced by the respective CD3 chain, and the (trispecific) functional CD3 domain is formed, while optionally an inactive dummy dissociates off.

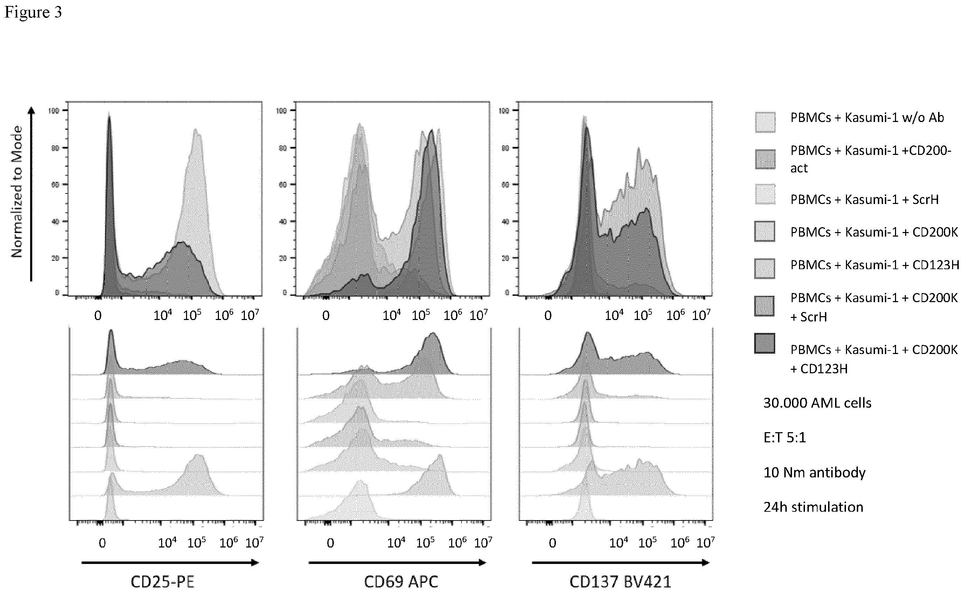

Figure 3: Flow cytometric analysis of PBMCs. PBMCs were incubated for 24 hours together with Kasumi-1 cells and antibodies on a 96-well plate. 30,000 Kasumi-1 cells were seeded out per well. The effector to target ratio was 5:1. Stimulation took place with 10 nM antibody. The FACS profiles show the expression of the activation markers CD25, CD69 and CD137 on cytotoxic CD8 T cells. The individual halves as well as a combination of CD200K and a non-binding half, scrambled-hole (SrcH) were used as controls.

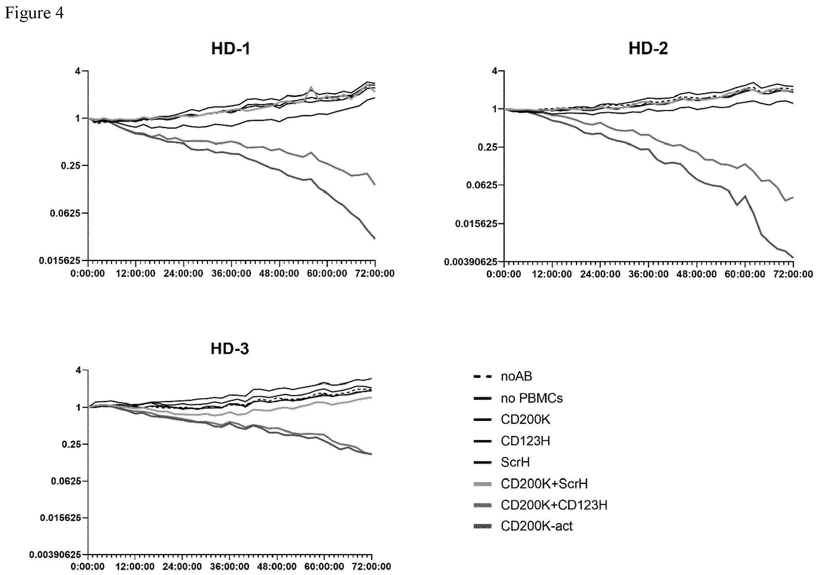

Figure 4: Killing curves of Kasumi-1mCherry cells. Kasumi-1-mCherry cells were incubated for a period of 72 h together with PBMCs from various healthy donors (HD1-3) and antibodies on a 96-well plate. 30,000 Kasumi-1 cells were seeded out per well. The effector to target ratio was 5: 1. Stimulation took place with 10 nM antibody. Every 2 hours a picture was taken in the Bright field and mCherry canal. The curves represent the relative increase/decrease of mCherry positive cells over time. Each curve is made up of three technical replicas. X-axis: t in [h]; Y-axis: log2 fold change of cell count/FOV.

SEQ ID NO: 1 shows the amino acid sequence of human CD123, isoform 1.

SEQ ID NO: 2 shows the amino acid sequence of human CD200, isoform 1.

SEQ ID NO: 3 shows the amino acid sequence of a light chain variable region (V_{L}) of an exemplary antigen binding domain binding to CD123.

SEQ ID NO: 4 shows the amino acid sequence of a heavy chain variable region (V_{H}) of an exemplary antigen binding domain binding to CD123.

SEQ ID NO: 5 shows the amino acid sequence of a light chain of an exemplary antigen binding domain binding to CD123.

SEQ ID NO: 6 shows the amino acid sequence of a heavy chain of an exemplary antigen binding domain binding to CD123.

SEQ ID NO: 7 shows the amino acid sequence of a heavy chain constant region of an exemplary antigen binding domain binding to CD123.

SEQ ID NOs: 8 to 13 show the amino acid sequences of CDRs of a first antigen binding domain binding to CD200.

SEQ ID NOs: 9 to 14 show the amino acid sequences of CDRs of a second antigen binding domain binding to CD200.

SEQ ID NOs: 15 to 20 show the amino acid sequences of CDRs of a third antigen binding domain binding to CD200.

SEQ ID NOs: 21 to 26 show the amino acid sequences of CDRs of a fourth antigen binding domain binding to CD200.

SEQ ID NOs: 27 to 32 show the amino acid sequences of CDRs of a fifth antigen binding domain binding to CD200.

### EXAMPLES

The results of in vitro experiments confirmed a selective binding to LSCs, and a controlled and improved and preferably localized activation of T-cells.

The exemplary trifab-contorsbody according to an embodiment of the invention was tested on the CD123⁺/CD200⁺ AML cell line Kasumi-1 (Asou H, Tashiro S, Hamamoto K, Otsuji A, Kita K, Kamada N. Establishment of a human acute myeloid leukemia cell line (Kasumi-1) with 8;21 chromosome translocation. Blood. 1991;77: 2031-2036). For this, the combination of anti-CD200-Knob (CD200K) with anti-CD123-hole (CD123H) was constructed. Furthermore, the individual halves as well as a combination of CD200K and a non-binding half, scrambled-hole (SrcH) were used as controls. One CD200K half having a functional anti-CD3 domain instead of a "dummy" served as positive control (CD200-act). (Figures 2 and 3)

In order to test to what extent the different combinations / individual halves of the constructs are able to activate T cells, PBMCs were isolated from healthy donors (HD) and cultured together with the antibodies and Kasumi-1 cells for 24 h. The T cells were then examined in a flow cytometer for the expression of the activation markers CD25, CD69 and CD137. There was a clearly increased expression of all three markers in case of PBMCs with Kasumi-1 together with CD200K + CD123H. In contrast, when PBMCs were co-cultivated with Kasumi-1 and the individual halves of the antibody, no activation was evident. The combination of CD200K + ScrH led to a slight increase in the CD69 activation marker, which, however, clearly lagged behind that from CD200K + CD123H. An increased CD25 and CD137 expression could not be determined with CD200K + ScrH. (Figure 4)

In the next step the inventors then investigated the cytotoxic activity of T cells depending on the TriFab exemplary construct with the help of a continuous visualization of the co-culture with mCherry-expressing Kasumi-1 cells in vitro. The co-culture was mixed with defined antibody halves in various combinations.

Over a period of 72 h, pictures were taken every 2 h in the bright field and mCherry channel. For Kasumi-1-mCherry together with PBMCs and the individual halves, an increase in mCherry positive cells was observed. For the combination of CD200K + CD123H, however, a clear eradication of the AML cells was found. The combination of CD200K + ScrH showed no difference to the control without antibodies.

Similar results were obtained with so-called "patient-derived xenograft leukemia cells", which are more heterogeneous than cell lines and are therefore biologically more comparable with primary leukemia cells [Vick B, Rothenberg M, Sandhöfer N, Carlet M, Finkenzeller C, Krupka C, et al. An advanced preclinical mouse model for acute myeloid leukemia using patients 'cells of various genetic subgroups and in vivo bioluminescence imaging. PLoS One. 2015; 10: e0120925].

The present in vitro experiments show that the invention can achieve selective cytotoxicity through a spatially and temporally controlled activation of T cells on the surface of CD123 + / CD200 + AML cells.

The combination of the two target structures thus constitutes a novel strategy to eliminate leukemic stem cells, in particular in pediatric forms of AML. The CD123 / CD200-dependent and advantageously localized activation of T cells is thus a principle that improves anti-AML immunotherapy.

## Claims

1. An in vitro method for identifying an at least bispecific antigen binding molecule that binds specifically to mammalian leukemic stem cells (LSCs), the method comprising the steps of:
a) providing a first library of candidate antigen binding molecules specific for the protein CD123 or immunologically recognizable fragments or derivatives thereof,
a') providing a second library of candidate antigen binding molecules specific for the protein CD200 or immunologically recognizable fragments or derivatives thereof,
b) combining a multitude of the candidate antigen binding molecules from the first and second library in order to produce a library of candidate molecules that are at least bispecific for CD123 and CD200,
c) providing a suitable screening system comprising the protein CD123 and/or CD200, or immunologically recognizable fragments or derivatives thereof,
d) contacting the library of b) with the screening system of c), and
e) identifying a binding molecule from the library of b) that specifically binds to the protein CD123 and CD200, or immunologically recognizable fragments or derivatives thereof, wherein preferably said LSCs are selected from LSC in acute myeloid leukemia (AML) or in chronic myeloid leukemia (CML).

2. The method according to claim 1, wherein the library of candidate antigen binding molecules is selected from an antibody library or a phage display library, preferably comprising single-chain variable fragments (scFvs) or stabilized form thereof (e.g., a disulfide stabilized scFv), Fab fragments, Fab' fragments, F(ab') fragments, F(ab')₂ fragments, scFabs, and single domain antibody fragments, such as heavy chain-only variable domain (VHH), Single-domain antibodies (sdAb), or nanobodies.

3. The method according to claim 1 or 2, wherein the library of candidate molecules that are at least bispecific binder for CD123 and CD200 comprises at least one bispecific format selected from the group consisting of TrioMab, IgG-like, CrossMab, 2:1 CrossMab, 2:2 CrossMab, DuoBody, DVD-Ig, scFv-IgG, IgG-IgG, Fab-scFv-Fc, TF, ADAPTIR, BITE, BITE-Fc, DART, DART-FC, Tetravalent DART, TandAb, ImmTAC, TriKE, scFv-scFv-scFv, Trispecific nanobody, a diabody, triabody, tetrabody or higher order multimer, and a trifab-contorsbody.

4. The method according to any one of claims 1 to 3, wherein the candidate molecules of the library further comprise at least one third antigen binding domain, for example a CD3 binding domain.

5. The method according to any one of claims 1 to 4, wherein the suitable screening system comprises a cell expressing, and in particular recombinantly expressing, CD123 and CD200 or antigenic fragments thereof, or is a cell-free system.

6. An at least bispecific antigen binding molecule that binds specifically to the proteins CD123 and CD200 or immunologically recognizable fragments or derivatives thereof of mammalian leukemic stem cells (LSCs), identified with a method according to any one of claims 1 to 5, wherein preferably said LSCs are selected from LSC in acute myeloid leukemia (AML) or in chronic myeloid leukaemia (CML).

7. The at least bispecific antigen binding molecule according to claim 6, wherein the amino acid sequence of the CD123 binding region is according to EP2778175A1 or specifically binding sequences that are at least 95% identical to the sequence, and wherein the amino acid sequence of the CD200 binding region is according to EP2178561A1 or specifically binding sequences that are at least 95% identical to the sequence.

8. The at least bispecific antigen binding molecule according to claim 6 or 7, which is murine, chimeric, humanized, or fully human.

9. A pharmaceutical composition, comprising the at least bispecific antigen binding molecule according to any one of claims 6 to 8, together with a pharmaceutically acceptable carrier and/or auxiliary agent.

10. The at least bispecific antigen binding molecule according to any one of claims 6 to 8 or the pharmaceutical composition according to claim 9, for use in medicine, preferably for use in the prevention or treatment of cancer in a patient, such as leukemia, for example AML or CML, and in particular pediatric forms thereof, comprising administering a effective amount of the at least bispecific antigen binding molecule according to any one of claims 6 to 8 or the pharmaceutical composition according to claim 9 to said patient.

11. The at least bispecific antigen binding molecule or the pharmaceutical composition for use according to claim 10, wherein said cancer prevention or treatment comprises immunotherapy and/or includes the avoidance of target loss and/or metastasis of said cancer.

12. A method for identifying mammalian leukemic stem cells (LSCs) in a biological sample obtained from a mammalian patient suspected to have leukemia, comprising
a) contacting the sample with the at least bispecific antigen binding molecule according to any one of claims 6 to 8, and
b) detecting binding of the at least bispecific antigen binding molecule to cells in said sample,
wherein a binding, and in particular a specific binding of the at least bispecific antigen binding molecule to the cells identifies leukemic stem cells (LSCs) in said sample.

13. The method for identifying mammalian leukemic stem cells (LSCs) in a biological sample according to claim 12, wherein the at least bispecific antigen binding molecule binds CD123 and CD200 simultaneously.

14. A method for diagnosing leukemia in a mammalian patient suspected to have leukemia, comprising a method according to claim 12 or 13, and further concluding on leukemia in the mammalian patient based on the binding, wherein preferably said leukemia is relapsing, malign, and/or metastasizing leukemia, AML, in particular pediatric form thereof, and CML.

15. Use of the at least bispecific antigen binding molecule according to any one of claims 6 to 8 to purify and/or isolate LSCs.
